# EUROPEAN PATENT SPECIFICATION

(11) **EP 1 828 779 B1**
(45) Date of publication and mention of the grant of the patent: **18.05.2011**
(21) Application number: 05850751.8
(22) Date of filing: 13.12.2005
(51) Int. Cl.: G01N 33/68, G01N 33/566

(54) **METHOD FOR IDENTIFYING MODULATORS OF THE ACTIVITY OF ION-CHANNELS RECEPTORS**
VERFAHREN ZUR IDENTIFIZIERUNG VON MODULATOREN DER AKTIVITÄT VON IONENKANALREZEPTOREN
NOUVEAU PROCEDE D'IDENTIFICATION DE MODULATEURS DE L'ACTIVITE DE RECEPTEURS A CANAUX IONIQUES

(30) Priority: 13.12.2004 US 635007 P
(43) Date of publication of application: 05.09.2007
(73) Proprietor: Centre National de la Recherche Scientifique - CNRS, 75016 Paris Cedex 16 (FR)
(72) Inventor: BOUE-GRABOT, Eric, F-33130 Begles (FR); CAZALETS, Jean-René, F-33800 Bordeaux (FR); TOULME, Estelle, F-33000 Bordeaux (FR)
(74) Representative: Warcoin, Jacques
(86) International application number: PCT/IB2005/004020
(87) International publication number: WO 2006/064372

(56) References cited:
- WO-A-99/55901
- SOKOLOVA ELENA ET AL: "Negative cross talk between anionic GABAA and cationic P2X ionotropic receptors of rat dorsal root ganglion neurons" JOURNAL OF NEUROSCIENCE, vol. 21, no. 14, 15 July 2001 (2001-07-15), pages 4958-4968, XP002380226 ISSN: 0270-6474
- BOUE-GRABOT E ET AL: "SUBUNIT-SPECIFIC COUPLING BETWEEN GAMMA-AMINOBUTYRIC ACID TYPE A AND P2X2 RECPETOR CHANNELS" JOURNAL OF BIOLOGICAL CHEMISTRY, AMERICAN SOCIETY OF BIOLOCHEMICAL BIOLOGISTS, BIRMINGHAM,, US, vol. 279, no. 50, 10 December 2004 (2004-12-10), pages 52517-52525, XP008063281 ISSN: 0021-9258 cited in the application
- BOUE-GRABOR E ET AL: "CROSS-TALK AND CO-TRAFFICKING BETWEEN RHO1/GABA RECEPTORS AND ATP-GATED CHANNELS" JOURNAL OF BIOLOGICAL CHEMISTRY, AMERICAN SOCIETY OF BIOLOCHEMICAL BIOLOGISTS, BIRMINGHAM,, US, vol. 279, no. 8, 20 February 2004 (2004-02-20), pages 6967-6975, XP008063282 ISSN: 0021-9258 cited in the application
- EMERIT M B ET AL: "ALTERED ADDRESSING OF GABA(C) RECEPTORS IN THE PRESENCE OF P2X2 RECEPTORS IN TRANSFECTED HIPPOCAMPAL NEURONS" SOCIETY FOR NEUROSCIENCE ABSTRACTS, SOCIETY FOR NEUROSCIENCE, US, vol. 2003, 8 November 2003 (2003-11-08), page ABSTRNR57010, XP008063293 ISSN: 0190-5295
- BHAGWAT SS WILLIAMS M: "P2 purine and pyrimidine receptors: emerging superfamilies of G-protein-coupled and ligand-gated ion channel receptors" EUROPEAN JOURNAL OF MEDICINAL CHEMISTRY, EDITIONS SCIENTIFIQUE ELSEVIER, PARIS, FR, vol. 32, no. 3, 1997, pages 183-193, XP004075420 ISSN: 0223-5234

## Description

The present invention is directed to a method for selecting or identifying a compound capable of modulating the activity of an ion-channels receptor, such as GABA receptor, by measuring the calcium influx via the P2X receptor in presence of its agonist ATP when coupled to said ion-channels receptor in cells recombinantly co-expressing these two receptors. The invention also concerns a kit or a device comprising the essentiel elements to perform the method according to the invention. Finally, the present invention is directed to the use of said selected compounds for the prevention or the treatment of diseases related to said ion-channels receptor dysfunction.

gamma-Aminobutyric acid (GABA) receptors are intrinsic membrane glycoproteins in mammal neuronal tissues that are members of the ligand-gated ion channel superfamily of receptors (named "ion-channels receptors" in the present description). GABA receptors play a major role in the inhibition of central nervous system (CNS) neuronal activity due to the widespread distribution of GABA-releasing and GABA-receptive neurons.

GABA receptors can be divided into two major classes: the GABA-A and GABA-C receptor subtypes, and GABA-B receptor types, which are distinguished by differences in their effector mechanisms and pharmacology. GABA-A and GABA-C receptors are transmitter-operated chloride channels that are activated by GABA to open their chloride channel.

GABA receptor channels, proteins which are present onto the surface of neurones, are the main inhibitory receptors of the central nervous system and are composed of five subunits that assemble to form a channel that is permeable to anions, mainly chloride ions.

There are 6 alpha subunit, 3 beta subunits, 3 gamma, 1 epsilon,1 delta, 1 pi and 3 rho subunits. It is thought that the majority of GABA-A receptors are formed by the association of 2 alpha subunits, 2 beta and 1 gamma, although other combinations are possible. All these combinations result in receptors with different properties. All these subunits have a similar topology, with a large extracellular domain, 4 transmembrane domains and one major intracellular domain connecting the M3 and M4 transmembrane domains.

The electric currents induce by the ions movements via these ion-channels receptor complexes are low and do not allow the direct use of fluorescent probe to quantify these chloride ions influx into the cells. The method and device allowing the electrophysiological recordings of these currents are complex. For example, it is also necessary to impose a constant voltage and to work on a single cell.

So, this method using electrophysiological recordings cannot be used to perform primary screening of compounds capable of modulating such receptor channels, compound which could be of a great interest in pharmaceutical field.

The P2X receptors are ligand-gated ion channels while the other "P2 receptor", P2Y receptors, operate generally through a G protein-coupled system. Several P2X receptor subtypes (P2X1- P2X7) form homomeric and heteromeric channels that are permeable to cations, including calcium, and which are specifically activated by extracellular ATP, its agonist. This receptor functions as a ligand-gated ion channel with relatively high calcium permeability (Egan et al., Neurosciences, 24(13):3413-3420, 2004). P2X receptors mediate membrane depolarization and Ca⁺⁺ influx and have physiological role ranging from fast excitatory synaptic transmission, pain reception to vessel vasoconstriction. Through the P2X receptors, ATP acts as an excitatory neurotransmitter in the central and peripheral nervous system.

Using electrophysiological recordings (i.e. by measuring the currents resulting from the passage of ions through the open channels), it has been already shown that GABA receptors and P2X receptors are functionally and physically coupled when they are co-expressed in a heterologous expression system (Xenopus oocytes, transfected mammalian cells or neurones expressing both receptor families) (Boué-Grabot et al., Journal of Biological Chemistry, 279:6967-6975, 2004; Boué-Grabot et al., Journal of Biological Chemistry, 279; 5251 52525, 2004).

We can also cite the document Sokolova et al. (J. of Neurosci. 2001, 21(14): 4958-68) which discloses the dependent interaction inhibition between the P2x3 and GABA (A) receptors expressed in the rat dorsal root ganglion neurons by an electrophysiology method.

It has been also shown that P2X interact also with nicotinic acethylcholine receptors and 5-HT3 receptors.

So, it is desirable to provide with an efficient method, no expensive and easy to perform, which can be used by high-throuhtput screening, for selecting compound capable of modulating the activity of ion-channels receptor such as GABA receptor.

This is the object of the present invention.

Surprisingly, the inventors have demonstrated that the activation of BABA-A receptors directly reduces the influx of calcium via opened P2X receptors, measured using a fluorescent calcium probe.

The inventors have also noticed by electrophysiological recordings that:
- when ATP and GABA are co-applied, the observed current is lower than the sum of the individual currents, demonstrating that the two receptors exhibit reciprocal inhibition when they are co-activated. This functional inhibition is based on the molecular coupling of the receptors at their respective intracellular domains. The inventors have also noticed that that the properties of each receptor are not altered by the presence of the other type of receptor. Thus, the presence of the P2X receptor does not modify the properties of the GABA receptor with respect to its agonist (GABA). The presence of the GABA receptor does not modify the properties of the P2X receptor with respect to its agonist (ATP). Only the amplitude of the response of each receptor is decreased when they are activated simultaneously,
- all the GABA receptors (GABA-A, irrespective of their molecular composition and GABA-C) inhibit the P2X2 receptors, such as P2X on co-activation;
- the responses of the P2X2 receptors to ATP are stable during application (they do not desensitise) and over time (good recovery). It can therefore be used to test many compounds rapidly using one single cell; and
- ATP-activated P2X receptors mediate a marked influx of calcium into a cell maintained at its resting potential.

Consequently, by co-expressing GABA and P2X receptors in a cell model, activation of the GABA receptors will be followed by modification of the fluorescence intensity of the response of the P2X receptors induced by ATP, or any of other well known P2X agonists.

So, in a first aspect, the present invention relates to a method for selecting or identifying a compound capable of modulating the activity of an ion-channels receptor, wherein said method comprises the following steps of:
a) having recombinant cells recombinantly expressing said ion-channels receptor and a P2X receptor;
b) contacting said recombinant cells with the compound to be tested in presence of an agonist of said P2X receptor, preferably such as ATP, or in presence of an agonist of said P2X receptor and the agonist of said ion-channels receptor;
c) measuring the calcium influx via the P2X receptor;
d) comparing the measure obtained at step c) with the measure obtained in the same conditions for a first reference control wherein in step b) the compound to be tested is not present; and
e) selecting said compound or identifying said compound as a compound capable of modulating the activity of an ion-channels receptor, if the comparison in step d) demonstrates a significant increase or decrease of the calcium influx in the recombinant cells in presence of said compound;

If the structure of the tested or selected compound is not initially known, its structure could be identified by the structure identification methods well known by the skilled man, such as mass or U.V. spectrometry, LC-MS, sequencing, ... after the step e) of the above method for selecting or identifying compound of the present invention.

In a preferred embodiment, the recombinant cells used in step a) do not express endogenously a receptor capable of being modulated by an agonist of said P2X receptor, preferably such as ATP, or by an agonist of said ion-channels receptor, such as GABA if said ion-channels receptor is GABA receptor.

In a more preferred embodiment, the recombinant cells used in step a) are oocytes, which do not endogenously express receptors activated by GABA or an agonist of said P2X receptor, preferably such as ATP.

Preferably, the recombinant cells used in step a) are mammal cells or Xenopus oocytes. It is more preferable that the cell model (mammalian cells or Xenopus oocytes) co-expresses the GABA receptor of interest and the P2X receptor in a stable or transient fashion.

Preferably, said mammal cells are human, from rat or mouse.

By P2X receptor, it is intended to designate in the present description, a natural P2X receptor which has as amino acid sequence the wild type amino acid sequence of a mammal P2X receptor or a mutated mammal P2X receptor capable of interacting with said ion-channels receptor.

Among said P2X receptor, are preferred the P2X receptor selecting from the group consisting of the P2X1, P2X2, P2X3, P2X4, P2X5, P2X6 or P2X7 receptor subunit or any P2X receptor which results from an association or a combination of P2X receptor subunits capable of exercising an ATP activated channel-receptor function.

It is believed that P2X receptors comprise multimers of receptor polypeptides, which multimers may be of either the same or different subtypes. Consequently, the term "P2X receptor" refers, as appropriate, to the individual receptor subunit or subunits, as well as to the homomeric and heteromeric receptors comprised thereby.

According to a likewise particular aspect, said ion-channels receptor or said P2X receptor is from human, rat or mouse source.

The amino acid (or mRNA) sequence of these subtypes of P2X receptor (also named P2RX) are well known by the skill person and could be found, for example, in Data Bank, such as in Genbank under the following Accession Number (see Table 1):

**Table 1: Examples of GenBank Accession Number depicted the complete sequence of P2X receptor subtypes, or splicing variants thereof, from rat, mouse and human source.**

| **P2XR subtypes** | **Rat** | **Mouse** | **Human** |
|---|---|---|---|
| **P2X1** | X80477 | NM008771 | AF020498; U45448; AF078925 |
| **P2X2** | U14414;NM_053656; AF013241; AF020759; AF020758; AF020757; AF020756; Y10473; Y10474; Y10475 | NM_153400; AB094664; AY044240; AB094663 | NM174873; NM174872; NM012226; NM170683; NM170682; NM016318 |
| **P2X3** | AF084975; X91167; X90651; NM_031075 | NM_145526 | Y07683; AB016608 |
| **P2X4** | X91200; U87270; U32497; X93565 | AF089751; AF089752; AF146516 | AF012903; U83993; Y07684; AF000234 |
| **P2X5** | X92069 ; X97328 | NM_033321 | U49395; AF016709; AF070573; U49396 |
| **P2X6** | X97376 ; X92070 | AB010883 | XM496501 |
| **P2X7** | NM_019256 | AJ009823 | Y09561; Y12851 |

By an agonist of P2X receptor, it intended to designate in the present description, one of the P2X receptor agonist well known by the skilled person, such as ATP, α,β mATP (alphabeta-methylene ATP), benzoylbenzoic ATP (such as 2' and 3' mixed isomers or 2',3'-O-(4-benzoylbenzoyl)-ATP (BzATP)), or 2-methylthio-ATP (2-MeSATP), (Zhao et al., J Pharmacol Exp Ther., 311(3):1211-7, 2004; Bianchi et al., Eur J Pharmacol. 2;376(1-2):127-38, 1999; Gendron et al., J Neurochem., 87(2):344-52, 2003), ATP being the more preferred agonist of P2X receptor.

In a preferred embodiment, said ion-channels receptor is selecting from the group consisting of the GABA receptor, the glycine receptor, the acetylcholin receptor or the serotonin receptor (5-HT3 receptor), the GABA receptor being the most preferred.

By ion-channels GABA receptor, it is intended to designate the ion-channels GABA receptor resulting from the homomeric or heteromeric association between any GABA receptor subunit, said subunit being selecting from the group of GABA receptor subunit consisting of alpha 1 to 6, beta 1 to 3, gamma 1 to 3, epsilon, delta, pi, theta, and rho 1 to 3 subunit, and said homomeric or heteromeric association forming an ion-channels receptor capable of being activated by GABA.

The amino acid (or mRNA) sequence of these GABA receptor subunits are well known by the skill person and could be found, for example, in Data Bank, such as in Genbank, under the following Accession Number (see Table 2):

**Table 2: Examples of GenBank Accession Number depicted the complete sequence of GABA receptor ("GABAR") subunit, or splicing variants thereof, from rat, mouse and human source.**

| **GABAR subunits** | **Rat** | **Mouse** | **Human** |
|---|---|---|---|
| **Alpha1** | L08490 | M86566; M57519 | X14766 |
| **Alpha2** | L08491 | M86567; M57520 | S62907 |
| **Alpha3** | L08492 | M86568; M57521 | S62908 |
| **Alpha4** | L08493 | | U30461 |
| **Alpha5** | L08494 | NM176942 | L08485 |
| **Alpha6** | L08495 | X51986 | S81944 |
| **Beta1** | X15466 | U14418; G755155 | X14767 |
| **Beta2** | X15467 | NM008070 | S67368 |
| **Beta3** | X15468 | NM008071 | M82919 |
| **Gamma1** | X57514 | NM010252 | |
| **Gamma2** | L08497 | M62374 | X15376 |
| **Gamma3** | X63324 | X59300 | AF269144 |
| **Epsilon** | NM023091; AF255612 | NM017369; AF18926 | Y07637 |
| **Delta** | L08496 | NM008072 | AF016917 |
| **theta** | NM031733 | AF189260; NM020488 | AF189259; AF14468 |
| **Pi** | | NM146017 | U95367 |
| **Rho1** | X95579; U21070 | NM008075 | M62400; M62323; |
| **Rho2** | D38494 | NM008076 | M86868 |
| **Rho3** | D50671 | XM484554 | |

When the ion-channels receptor is the GABA receptor, the agonist of said ion-channels receptor is GABA or any of other well known GABA receptor agonist, such as muscimol, isoguvacine.

In the method according to the present invention, the calcium influx measured at step c) is measured by a fluorescent calcium probe, such as calcium green, Fluo-3 or Fluo-4, fluorescent calcium probe.

In a preferred embodiment, said compound to be tested is tested for its ability to interact specifically with said P2X receptor. Said specific interacting can be demonstrated by measuring whether a significant increase or decrease of the calcium influx is obtained in the same conditions for a second reference control wherein in step a) the recombinant cells used for said second reference control express said P2X receptor and do not express said ion-channels receptor.

Particularly, said compound to be tested will be not selected if a significant increase or decrease of the calcium influx is observed in the recombinant cells for said second reference control.

In a second aspect, the present invention relates to a method for selecting or identifying an agonist of an ion-channels receptor, said method comprising the following steps of:
A) selecting a compound capable of modulating the activity of said ion-channels receptor by a method for selecting or identifying a compound capable of modulating the activity of an ion-channels receptor according to the invention, wherein in step b) said recombinant cells are contacted with the compound to be tested only in presence of an agonist of said P2X receptor, such as ATP; and
B) the selection of said compound as an agonist if the comparison carried out in step d) of the method according to the present invention demonstrates a significant decrease of the calcium influx is observed in the recombinant cells in presence of said compound compared to the results obtained for the first reference control.

It also preferred a method for selecting or identifying a positive modulator of an ion-channels receptor, said method comprising the following steps of:
A) selecting or identifying a compound capable of modulating the activity of said ion-channels receptor by a method for selecting or identifying a compound capable of modulating the activity of an ion-channels receptor according to the present invention wherein in step b) said recombinant cells are contacted with the compound to be tested in presence of an agonist of said P2X receptor, such as ATP, and the agonist of said ion-channels receptor; and
B) the selection of said compound as a posive modulator if the comparison carried out in step d) of the method according to the present invention demonstrates a significant decrease of the calcium influx is observed in the recombinant cells in presence of said compound compared to the results obtained for the first reference control.

In a more preferred embodiment, in the method for selecting or identifying a posive modulator of an ion-channels receptor according to the present invention, the agonist of the ion-channels receptor is used at a non-saturated concentration in step b) of the method for selecting or identifying a compound capable of modulating the activity of an ion-channels receptor according to the present invention.

In a third aspect, the present invention relates to a method for selecting or identifying a negative modulator, an inhibitor or an antagonist of an ion-channels receptor, said method comprising the following steps of:
A) selecting or identifying a compound capable of modulating the activity of said ion-channels receptor by a method for selecting or identifying a compound capable of modulating the activity of an ion-channels receptor according to the present invention wherein in step b) said recombinant cells are contacted with the compound to be tested in presence of an agonist of said P2X receptor, such as ATP, and the agonist of said ion-channels receptor; and
B) selecting said compound as a positive modulator if the comparison carried out in step d) of the method according to the present invention demonstrates a significant increase of the calcium influx is observed in the recombinant cells in presence of said compound compared to the results obtained for the first reference control.

In a fourth aspect, the present invention is also directed to a method for selecting or identifying a compound capable of modulating the activity of an ion-channels receptor, an agonist a positive or negative modulator, according to the present invention wherein the same recombinants cells used for testing a first compound can be used for testing at least a second compound to be tested. Indeed, the responses of the P2X2 receptors to its agonist, such as ATP, are stable during application (they do not desensitise) and over time (good recover). It can therefore be used to test many compounds rapidly using one single cell.

In a fifth aspect, the present invention concerns a kit for the selection of a compound capable of modulating the activity of an ion-channels receptor, wherein said kit comprises:
- cells which recombinantly co-expressing an ion-channels receptor and a P2X receptor, or, optionally, means to obtain such recombinant cells;
- ATP or any of other P2XR agonist such as α,β mATP (alphabeta-methylene ATP), benzoylbenzoic ATP (such as 2' and 3' mixed isomers or 2',3'-O-(4-benzoylbenzoyl)-ATP (BzATP)), or 2-methylthio-ATP (2-MeSATP);
- optionally, an agonist of said ion-channels receptor, such as GABA;
- optionally, a calcium probe, preferably a fluorescent calcium probe, as a marker for measuring the calcium influx in said recombinant cells; and
- optionally, a system to measure fluorescence such as Video microscopy or Fluorescence Image Plate Recorder (FCIPR) assay.

In the kit according to the present invention, said ion-channels receptor, P2X receptor, recombinant cells and calcium probe are independently chosen among those as defined above for the method of the invention for selecting or identifying a compound capable of modulating the activity of an ion-channels receptor.

It is also described a device or system, such system which can be used by high-throughput screening (HTS) for the selection of a compound capable of modulating the activity of an ion-channels receptor, wherein said device comprises the essential elements as defined above for the method or the kit of the invention for selecting a compound capable of modulating the activity of an ion-channels receptor.

This invention can be used to select compounds capable of activating, modulating or inhibiting ion-channels receptor, such as GABA receptors, functionally coupled with P2X receptor, by measuring calcium indicators, particularly with a fluorescent calcium probe capable of measured the calcium influx via the P2X receptor.

In a final aspect, it is described new compound or compound selected or identified by the methods for selecting or for identifying according to the invention as defined above, these new compounds or already known compounds being newly identified as modulator, such as agonist, positive or negative modulator, of the activity of an ion-channels receptor, said ion-channels receptor being preferably a receptor capable of interacting functionally with P2X receptors, such as GABA, glycine, serotonin and acetylcholine receptors.

Said new compounds or already known compounds are compounds being newly identified as modulator, pos of the activity of GABA receptor, particularly the GABA-A receptor, and are selected or identified by the method according to the present invention, wherein in said method, said ion-channels receptor is a GABA receptor, particular the GABA-A receptor.

It is also disclosed the use of a said compound for the diagnosis, prevention and treatment of diseases and disorders in mammals, including man, which are related to ion-channels receptor which can interact functionally with P2X receptors.

This compound can be used for the prevention or the treatment, or for the manufacture of a medicament for the prevention or the treatment, of diseases, disorders or condition in mammals, including man, which are related to ion-channels receptor which can interact functionally with P2X receptors. More preferably, said disease, disorder or condition is related to GABA-A receptor dysfunction.

When said disease, disorder or condition is related to GABA receptor dysfunction, particularly GABA-A dysfunction, preferred are a disease, disorder or condition selected from the group consisting of asthma, acute heart failure, hypotension, urinary retention, osteoporosis, hypertension, vagina pectoris, myocardial infraction, ulcers, allergies, benign prostatic hypertrophy, prostate cancer, Parkinson's disease, psychotic and neurological disorders, anxiety, schizophrenia, mania, depression, dyskinesia, memory disorders, sleep disorders, convulsive disorders, or epilepsy.

These new compounds identified using the present invention will be useful in the diagnosis, prevention and treatment of diseases and disorders in mammals, including man, Concerning particularly the GABA receptor, these new compounds identified using the present invention will be useful in the diagnosis, prevention and treatment of diseases and disorders such as dysfunction: asthma, hypotension, hypertension, urinary retention, angina, myocardial infarction, ulcers, allergies, Parkinson's disease, neurological disorders, anxiety, schizophrenia, depression, dyskinesia, cognitive disorders, sleep disorders, convulsive disorders and epilepsy.

A test compound identified using the methods of the invention, or a pharmaceutically acceptable salt thereof, is administered to a patient, preferably a mammal, more preferably a human, suffering from a disease whose progression is associated with a lack or a too much permeability of the ion-channels formed by the ion-channels receptor which can interact functionally with P2X receptors, such as GABA, glycine, serotonin and acetylcholine receptors. As used therein, "treatment" refers to an amelioration of a disease, or at least one discernible symptom thereof or to an amelioration of at least one measurably physical parameter, not necessarily discernible by the patient or to delaying the onset of the disease. As used herein, "prevention" refers to a reduction of the risk of acquiring a disease. The patient can have a genetic predisposition to a disease, such as a family history of the disease, or a non-genetic predisposition to the disease.

When administered to a patient, a test compound or a pharmaceutically acceptable salt thereof is preferably administered as component of a composition that optionally comprises a pharmaceutically acceptable vehicle. The composition can be administered orally, or by any other convenient route, and may be administered together with another biologically active agent. Administration can be systemic or local. Various delivery systems are known, e.g., encapsulation in liposomes, microparticles, microcapsules, capsules, etc., and can be used to administer the selected compound of the present invention or pharmaceutically acceptable salts thereof

Methods of administration include but are not limited to intradermal, intramuscular, intraperitoneal, intravenous, subcutaneous, intranasal, epidural, oral, sublingual, intranasal, intracerebral, intravaginal, transdermal, rectally, by inhalation, or topically. The mode of anon is left to the discretion of the practitioner. In most instances, administration will result in the release of a test compound or a pharmaceutically acceptable salt thereof into the bloodstream.

Compositions comprising a test compound selected by the methods according to the present invention, or a pharmaceutically acceptable salt thereof, can additionally comprise a suitable amount of a pharmaceutically acceptable vehicle so as to provide the form for proper administration to the patient. The term "pharmaceutically acceptable" means approved by a regulatory agency or listed by a national or a recognized pharmacopeia for use in animals, mammals, and more particularly in humans. The term "vehicle" refers to a diluent, adjuvant, excipient, or carrier with which a compound is administered. Such pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gelatin, starch and the like. In addition, auxiliary, stabilizing, thickening, lubricating and coloring agents may be used. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, sodium stearate, glycerol monostearate, sodium chloride, dried skim milk, glycerol, propylene, glycol, water and the like. Test compound compositions, if desired, can also contain minor amounts of wetting or emulsifying agents, or pH buffering agents. The compositions of the invention comprising the selected compound can take the form of solutions, suspensions, emulsion, tablets, pills, pellets, capsules, capsules containing liquids, powders, sustained-release formulations, suppositories, emulsions, aerosols, sprays, suspensions, or any other form suitable for use. Said composition is generally formulated in accordance with routine procedures as a pharmaceutical composition adapted to human beings for oral administration or for intravenous administration. The amount of the selected compound or a pharmaceutically acceptable salt thereof that will be effective in the treatment of a particular disease will depend on the nature of the disease, and can be determined by standard clinical techniques. In addition, in vitro or in vivo assays may optionally be employed to help identify optimal dosage ranges. The precise dose to be employed will also depend on the route of administration, and the seriousness of the disease, and should be decided according to the judgment of the practitioner and each patient's circumstances. However, suitable dosage ranges for oral, intranasal, intradermal or intraveneous administration are generally about 0.01 milligram to about 75 milligrams per kilogram body weight per day, more preferably about 0.5 milligram to 5 milligrams per kilogram body weight per day.

Other characteristics and advantages of the invention appear in the continuation of the description with the examples and the figures whose legends are represented below.

### BRIEF DESCRIPTION OF THE FIGURES

**Figure 1****:** Fluorescence intensity measured after application of ATP, or GABA onto an oocyte not expressing P2X and GABA receptors.
**Figure 2****:** Fluorescence intensity measured after application of ATP+GABA, GABA or ATP, onto an oocyte expressing the P2X2 receptor.
**Figure 3****:** Fluorescence intensity measured after application of ATP+GABA, ATP or GABA, onto an oocyte co-expressing the GABA-A receptor and P2X2 receptor.
**Figure 4****:** Fluorescence intensity measured after application of GABA at different moment of the ATP response, onto an oocyte co-expressing the GABA-A receptor and P2X2 receptor.

### EXAMPLE

### Example 1: Material and Methods

Using an automatic nano injector, cDNA encoding rat P2X2 receptors and/or GABA receptors composed for example of rat a2 and rat b3 subunits have been injected into the nucleus of Xenopus oocytes as previously described in Boué-Grabot et al., 2004 (Journal of Biological Chemistry, 279; 52517-52525, 2004).

1 to 3 days after infection, the calcium green fluorescence probe, which is sensitive to the concentration of free intracellular calcium, was injected into oocytes expressing either P2X2 receptors alone, P2X and GABA receptors or no receptors (not injected). Then, using video-microscopy (Nikon), the fluorescence emitted by oocytes maintained in a standard solution (Ringer solution containing 1.8 mM calcium chloride), perfused at 3 ml/min, has been recorded. The substances (ATP 100 mM, GABA 100 µM, ATP +GABA 100 µM of each) diluted in the same buffer were applied using an application system (BPS8-AlaScientific). Variations in fluorescence were analysed using IpLab software.

### Example 2: Oocytes expressing no receptors

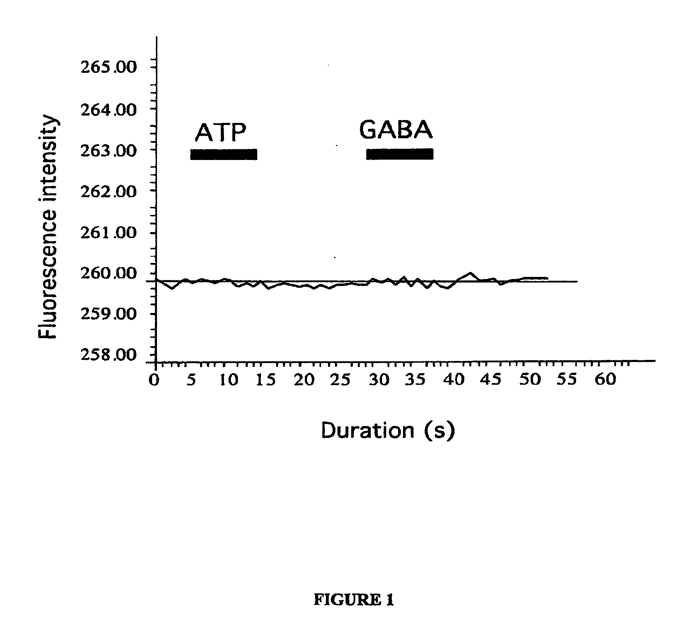

No modification of the fluorescence was observed on application of ATP or GABA onto an oocyte not expressing P2X and GABA receptors (Figure 1). These experiments confirm that the oocytes do not express endogenous receptors that are sensitive to ATP or GABA.

### Example 3: Recording from oocytes co-expressing P2X2 receptors alone

The application of ATP induces a transient increase in fluorescence that demonstrates the influx of calcium due to opening of the P2X2 receptor channels. GABA had no effect on the fluorescence, indicating that GABA does not activated P2X2 receptors. The co-application of ATP+GABA also results in an increase in fluorescence similar to that obtained with ATP alone.

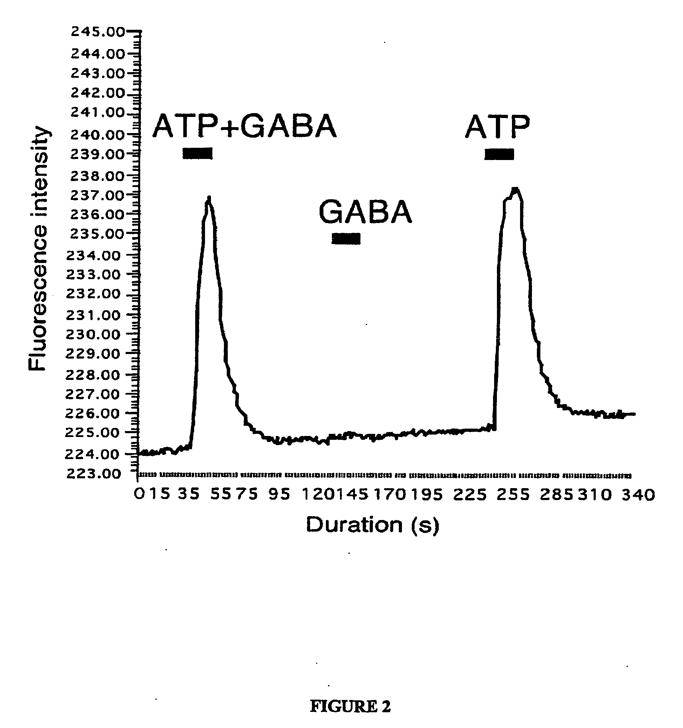

The increase in fluorescence induced by application of ATP onto oocytes expressing only P2X2 receptors is not altered by GABA (see Figure 2).

### Example 4: Recording of oocytes co-expressing P2X2 and GABA-A receptors

An application of ATP (100 µM) induces a marked transient increase in the fluorescence of the calcium probe, demonstrating activation of the P2X receptors and the subsequent influx of calcium (Figure 2). Successive applications of ATP induce responses of similar amplitude. It is important to note that this increase in fluorescence is observed at the cell's resting potential.

Application of GABA alone (100 µM) does not modify fluorescence intensity. This can be explained by the fact that activation of the GABA receptors causes a chloride channel to open, which has no effect on intracellular calcium concentration.

If a solution of ATP + GABA (100 µM of each) is co-applied, we obtain a lower calcium response than that obtained by applying ATP alone, thus showing that the opening of the GABA channels inhibits the calcium influx mediated by P2X receptors.

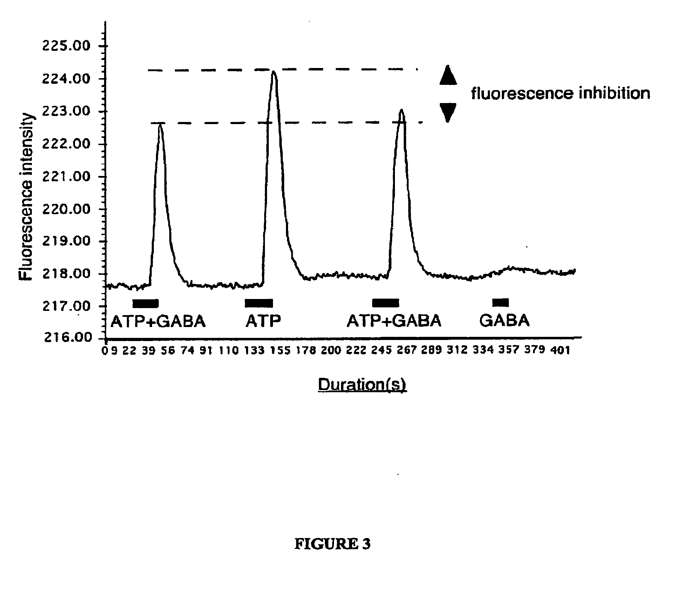

This decrease in fluorescence shows the activation of the GABA-A receptors (Figure 3).

The percentage inhibition obtained (20-25 % in these experiments) is a reflection of the expression ratio of the P2X and GABA receptors. As we have shown using electrophysiological techniques, if the ratio of P2X2 to GABA receptors is 1:1, the current inhibition by GABA of the response mediated by ATP can then reach 50 %. By calcium imaging, only opening of P2X channels is measured. So it can be postulate that if GABA-A receptors interact with expressed P2X2 receptors, activation of GABA receptors could lead to the closure of all P2X receptor.

### Example 5: Recording of the activation or inhibition of GABA receptors during the activation of P2X receptors of oocytes co-expressing P2X2 and GALA-A receptors

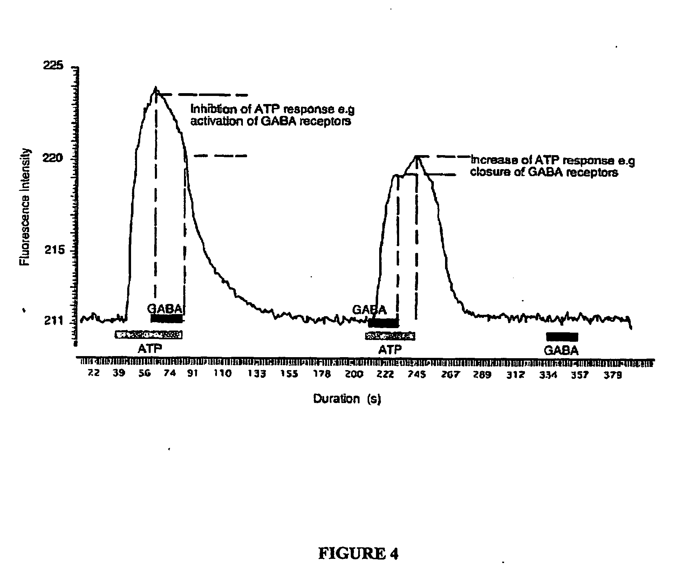

The activation or inhibition of GABA receptors can also be monitored during the activation of P2X receptors (see Figure 4).

If GABA is applied at the peak of the ATP response, a decrease in fluorescence intensity is observed, showing that when the GABA receptors are opened it leads to closure of the P2X receptors that were already open. And likewise, if during co-application of ATP and GABA, the application of GABA is stopped, an increase in fluorescence is observed. This indicates that closure of the GABA receptors lifts the inhibition of some of the P2X receptors, i.e. allows the P2X receptors to open (Figure 4).

This shows that irrespective of the sequence in which the agonists are applied, opening or closure of the GABA-A receptors directly modulates the calcium current (measured by the fluorescence of the calcium-sensitive probe) of the P2X channels induced by ATP. The measurement of calcium influx from the P2X receptors therefore represents a new procedure to monitor the activity of GABA receptors.

These experiments show the co-expression of rat GABA-A alpha2 beta3 and P2X2 receptors, but the present invention can be used to screen all rat, mouse or human GABA-A receptors using the P2X2 receptor or any other wild-type or mutated P2X receptors that retain the ability to interact with GABA receptors.

## Claims

1. Method for selecting or identifying a compound capable of modulating the activity of an ion-channels receptor, wherein said method comprises the following steps of:
a) having cells recombinantly co-expressing said ion-channels receptor and a P2X receptor;
b) contacting said recombinant cells with the compound to be tested in presence of an agonist of said P2X receptor, or in presence of an agonist of said P2X receptor and the agonist of said ion-channels receptor;
c) measuring the calcium influx via the P2X receptor, and wherein the calcium influx is measured by a calcium probe;
d) comparing the measure obtained at step c) with the measure obtained in the same conditions for a first reference control wherein in step b) the compound to be tested is not present; and
e) selecting said compound if the comparison in step d) demonstrates a significantly increase or decrease of the calcium influx in the recombinant cells in presence of said compound.

2. The method according to claim 1, wherein in step c) the calcium influx is measured by a fluorescent calcium probe.

3. The method according to claim 1 or 2, wherein in step c) the calcium influx is measured by a calcium green, Fluo-3 or Fluo-4, fluorescent calcium probe.

4. The method according to claim 1, wherein at step a) said recombinant cells do not express endogenously a receptor capable of being modulated by said agonist of said P2X receptor or by the agonist of said ion-channels receptor.

5. The method according to one of claims 1 to 4, wherein said recombinant cells in step a) are mammal cells or Xenopus oocytes.

6. The method according to one of claims 1 to 5, wherein said P2X receptor is a natural P2X receptor or a mutated P2X receptor capable of interacting with said ion-channels receptor.

7. The method according to claim 6, wherein said P2X receptor is selected from the group consisting of the P2X1, P2X2, P2X3, P2X4, P2X5, P2X6 or P2X7 receptor or any P2X receptor which results from the association or the combination of P2X subunit receptor capable of exercising an ATP activated channel-receptor function.

8. The method according to one of claims 1 to 7, wherein said ion-channels receptor or said P2X receptor is from human, rat or mouse source.

9. The method according to one of claims 1 to 8, wherein said agonist of said P2X receptor is selected from the group consisting of ATP, α,β mATP (alphabeta-methylene ATP), benzoylbenzoic ATP (such as 2' and 3' mixed isomers or 2',3'-O-(4-benzoylbenzoyl)-ATP (BzATP)), or 2-methylthio-ATP (2-MeSATP), preferably ATP.

10. The method according to one of claims 1 to 9, wherein said compound to be tested is tested for its ability to interact specifically with said P2X receptor, preferably said specific interacting being demonstrated by measuring whether a significant increase or decrease of the calcium influx is obtained in the same conditions for a second reference control wherein in step a) the recombinant cells used for said second reference control express said P2X receptor and do not express said ion-channels receptor.

11. The method according to claim 10, wherein said compound to be tested is not selected if a significant increase or decrease of the calcium influx is observed in the recombinant cells for said second reference control.

12. Method for selecting or identifying an agonist of an ion-channels receptor, said method comprising the following steps of:
A) selecting or identifying a compound capable of modulating the activity of said ion-channels receptor by a method according to one of claims 1 to 11 wherein in step b) said recombinant cells are contacted with the compound to be tested only in presence of an agonist of said P2X receptor; and
B) the selection of said compound as an agonist if the comparison carried out in step d) of the method according to one of claims 1 to 11 demonstrates a significant decrease of the calcium influx in the recombinant cells in presence of said compound compared to the results obtained for the first reference control.

13. Method for selecting a positive modulator of an ion-channels receptor, said method comprising the following steps of:
A) selecting or identifying a compound capable of modulating the activity of said ion-channels receptor by a method according to one of claims 1 to 11 wherein in step b) said recombinant cells are contacted with the compound to be tested in presence of an agonist of said P2X receptor and the agonist of said ion-channels receptor; and
B) the selection of said compound as a positive modulator if the comparison carried out in step d) of the method according to one of claims 1 to 11 demonstrates a significant decrease of the calcium influx in the recombinant cells in presence of said compound compared to the results obtained for the first reference control.

14. Method for selecting a positive modulator of an ion-channels receptor according to claim 13, wherein in step b) of the method according to one of claims 1 to 11 in A), the agonist of said ion-channels receptor is used at a non-saturated concentration.

15. Method for selecting or identifying a negative modulator, an inhibitor or an antagonist of an ion-channels receptor, said method comprising the following steps of:
A) selecting a compound capable of modulating the activity of said ion-channels receptor by a method according to one of claims 1 to 11 wherein in step b) said recombinant cells are contacted with the compound to be tested in presence of an agonist of said P2X receptor and the agonist of said ion-channels receptor; and
B) the selection of said compound as a negative modulator if the comparison carried out in step d) of the method according to one of claims 1 to 11 demonstrates a significant increase of the calcium influx in the recombinant cells in presence of said compound compared to the results obtained for the first reference control.

16. The method according to one of claims 1 to 15, wherein the same recombinant cells used for testing a first compound can be used for testing at least a second compound to be tested.

17. The method according to one of claims 1 to 16, wherein said ion-channels receptor is selected from the group consisting of the GABA receptor, the glycine receptor, the acetylcholine receptor or the serotonin receptor.

18. The method according to claim 17, wherein said ion-channels receptor is the GABA receptor.

19. The method according to claim 17 or 18, wherein said ion-channels receptor is a ion-channels GABA receptor resulting from the homomeric or heteromeric association between any GABA receptor subunit, said subunit being selected from the group of GABA receptor subunit consisting of alpha 1 to 6, beta 1 to 3, gamma 1 to 3, delta, epsilon, theta, pi, and rho 1 to 3 subunit, and said homomeric or heteromeric association forming an ion-channels receptor capable of being activated by GABA.

20. The method according to claim 18 or 19, wherein the agonist of said ion-channels receptor is GABA or any of other well known GABA receptor agonist, such as muscimol, isoguvacine.

21. Kit for the selection of a compound capable of modulating the activity of an ion-channels receptor, wherein said kit comprises:
- cells which recombinantly co-express an ion-channels receptor and a P2X receptor;
- an agonist of P2X receptor selected from the group consisting of ATP, α,β mATP (alphabeta-methylene ATP), benzoylbenzoic ATP (such as 2' and 3' mixed isomers or 2',3'-O-(4-benzoylbenzoyl)-ATP (BzATP)), or 2-methylthio-ATP (2-MeSATP), preferably ATP; and
- a calcium probe as a marker for measuring the calcium influx in said recombinant cells.

22. Kit according to claim 21, wherein said calcium probe is a fluorescent calcium probe.

23. Kit according to claim 21 or 22, wherein said kit further comprises an agonist of said ion-channels receptor.

24. Kit according to claim 23, wherein said agonist of said ion-channels receptor is GABA.

25. Kit according to claims 21 to 24, wherein said ion-channels receptor, P2X receptor, recombinant cells and calcium probe are independently chosen among those as defined in claims 1 to 20.

## Patentansprüche

1. Verfahren zum Auswählen oder Identifizieren einer Verbindung, welche fähig ist, die Aktivität eines Ionenkanal-Rezeptors zu modulieren, wobei das Verfahren die folgenden Schritte umfasst:
a) rekombinante Coexpression des Ionenkanal-Rezeptors und eines P2X-Rezeptors durch Zellen;
b) Inkontaktbringen der rekombinanten Zellen mit der zu testenden Verbindung in Gegenwart eines Agonisten des P2X-Rezeptors oder in Gegenwart eines Agonisten des P2X-Rezeptors und des Agonisten des lonenkanal-Rezeptors;
c) Messen des Calcium-Einstroms über den P2X-Rezeptor, und wobei der Calcium-Einstrom durch eine Calciumsonde gemessen wird;
d) Vergleichen der Messung wie in Schritt c) erhalten mit der Messung, welche unter den gleichen Bedingungen für eine erste Referenzkontrolle erhalten wurde, wobei in Schritt b) die zu testende Verbindung nicht vorhanden ist; und
e) Auswählen der Verbindung, wenn der Vergleich in Schritt d) eine signifikante Zunahme oder Abnahme des Calcium-Einstroms in den rekombinanten Zellen in Gegenwart der Verbindung zeigt.

2. Verfahren gemäß Anspruch 1, wobei in Schritt c) der Calcium-Einstrom durch eine fluoreszierende Calciumsonde gemessen wird.

3. Verfahren gemäß Anspruch 1 oder 2, wobei in Schritt c) der Calcium-Einstrom durch ein Calcium-Grün, Fluo-3 oder Fluo-4 als fluoreszierende Calciumsonde gemessen wird.

4. Verfahren gemäß Anspruch 1, wobei in Schritt a) die rekombinanten Zellen endogen keinen Rezeptor exprimieren, welcher durch den Agonisten des P2X-Rezeptors oder durch den Agonisten des Ionenkanalrezeptors moduliert werden kann.

5. Verfahren gemäß einem der Ansprüche 1 bis 4, wobei die rekombinanten Zellen in Schritt a) Säugerzellen oder Xenopus-Eizellen sind.

6. Verfahren gemäß einem der Ansprüche 1 bis 5, wobei der P2X-Rezeptor ein natürlicher P2X-Rezeptor oder ein mutierter P2X-Rezeptor ist, welcher fähig ist, mit dem Ionenkanal-Rezeptor zu interagieren.

7. Verfahren gemäß Anspruch 6, wobei der P2X-Rezeptor aus der Gruppe bestehend aus dem P2X1-, P2X2-, P2X3-, P2X4-, P2X5-, P2X6- oder P2X7-Rezeptor oder einem beliebigen P2X-Rezeptor ausgewählt ist, der aus der Assoziation oder Kombination von Untereinheiten des P2X-Rezeptors, welche fähig sind, eine ATPaktivierende Kanal-Rezeptor-Funktion auszuüben, resultiert.

8. Verfahren gemäß einem der Ansprüche 1 bis 7, wobei der Ionenkanal-Rezeptor oder der P2X-Rezeptor von Mensch-, Ratte- oder Maus-Ursprung ist.

9. Verfahren gemäß einem der Ansprüche 1 bis 8, wobei der Agonist des P2X-Rezeptors ausgewählt ist aus der Gruppe bestehend aus ATP, α,β-mATP (alphabeta-Methylen-ATP), Benzoylbenzoe-ATP (wie 2'- und 3'-gemischte Isomere oder 2',3'-O-(4-Benzoylbenzoyl)-ATP (BzATP)) oder 2-Methylthio-ATP (2-MeSATP), vorzugsweise ATP.

10. Verfahren gemäß einem der Ansprüche 1 bis 9, wobei die zu testende Verbindung bezüglich ihrer Fähigkeit getestet wird, spezifisch mit dem P2X-Rezeptor zu interagieren, wobei die spezifische Interaktion vorzugsweise gezeigt wird durch Messen, ob eine signifikante Zunahme oder Abnahme des Calcium-Einstroms unter den gleichen Bedingungen für eine zweite Referenzkontrolle erhalten wird, wobei in Schritt a) die rekombinanten Zellen, welche für die zweite Referenzkontrolle verwendet werden, den P2X-Rezeptor und nicht den Ionenkanal-Rezeptor exprimieren.

11. Verfahren gemäß Anspruch 10, wobei die zu testenden Verbindung nicht ausgewählt wird, wenn eine signifikante Zunahme oder Abnahme des Calcium-Einstroms in den rekombinanten Zellen für die zweite Referenzkontrolle beobachtet wird.

12. Verfahren zum Auswählen oder Identifizieren eines Agonisten eines Ionenkanal-Rezeptors, wobei das Verfahren die folgenden Schritte umfasst:
A) Auswählen oder Identifizieren einer Verbindung, welche fähig ist, die Aktivität des Ionenkanal-Rezeptors durch ein Verfahren gemäß einem der Ansprüche 1 bis 11 zu modulieren, wobei in Schritt b) die rekombinanten Zellen mit der zu testenden Verbindung nur in Gegenwart eines Agonisten des P2X-Rezeptors in Kontakt gebracht werden; und
B) die Auswahl der Verbindung als einen Agonisten, wenn der Vergleich, welcher in Schritt d) des Verfahrens gemäß einem der Ansprüche 1 bis 11 durchgeführt wird, eine signifikante Abnahme des Calcium-Einstroms in den rekombinanten Zellen in Gegenwart der Verbindung zeigt, im Vergleich zu den Ergebnissen, welche für die erste Referenzkontrolle erhalten wurden.

13. Verfahren zur Auswahl eines positiven Modulators eines Ionenkanal-Rezeptors, wobei das Verfahren die folgenden Schritte umfasst:
A) Auswählen oder Identifizieren einer Verbindung, welche fähig ist, die Aktivität des Ionenkanal-Rezeptors durch ein Verfahren gemäß einem der Ansprüche 1 bis 11 zu modulieren, wobei in Schritt b) die rekombinanten Zellen mit der zu testenden Verbindung in Gegenwart eines Agonisten des P2X-Rezeptors und des Agonisten des Ionenkanal-Rezeptors in Kontakt gebracht werden; und
B) die Auswahl der Verbindung als einen positiven Modulator, wenn der Vergleich, welcher in Schritt d) des Verfahrens gemäß einem der Ansprüche 1 bis 11 durchgeführt wird, eine signifikante Abnahme des Calcium-Einstroms in den rekombinanten Zellen in Gegenwart der Verbindung zeigt, im Vergleich zu den Ergebnissen, welche für die erste Referenzkontrolle erhalten wurden.

14. Verfahren zum Auswählen eines positiven Modulators eines Ionenkanal-Rezeptors gemäß Anspruch 13, wobei in Schritt b) des Verfahrens gemäß einem der Ansprüche 1 bis 11 in A) der Agonist des Ionenkanal-Rezeptors in einer nicht-gesättigten Konzentration verwendet wird.

15. Verfahren zum Auswählen oder Identifizieren eines negativen Modulators, eines Inhibitors oder eines Antagonisten eines Ionenkanal-Rezeptors, wobei das Verfahren die folgenden Schritte umfasst:
A) Auswählen einer Verbindung, welche fähig ist, die Aktivität des Ionenkanal-Rezeptors durch ein Verfahren gemäß einem der Ansprüche 1 bis 11 zu modulieren, wobei in Schritt b) die rekombinanten Zellen mit der zu testenden Verbindung in Gegenwart eines Agonisten des P2X-Rezeptors und des Agonisten des Ionenkanal-Rezeptors in Kontakt gebracht werden; und
B) die Auswahl der Verbindung als einen negativen Modulator, wenn der Vergleich, welcher in Schritt d) des Verfahrens gemäß einem der Ansprüche 1 bis 11 durchgeführt wird, eine signifikante Zunahme des Calcium-Einstroms in den rekombinanten Zellen in Gegenwart der Verbindung zeigt, im Vergleich zu den Ergebnissen, welche für die erste Referenzkontrolle erhalten wurden.

16. Verfahren gemäß einem der Ansprüche 1 bis 15, wobei die gleichen rekombinanten Zellen, die zum Testen einer ersten Verbindung verwendet werden, zum Testen mindestens einer zweiten zu testenden Verbindung verwendet werden können.

17. Verfahren gemäß einem der Ansprüche 1 bis 16, wobei der Ionenkanal-Rezeptor ausgewählt ist aus der Gruppe bestehend aus dem GABA-Rezeptor, dem Glycin-Rezeptor, dem Acetylcholin-Rezeptor oder dem Serotonin-Rezeptor.

18. Verfahren gemäß Anspruch 17, wobei der Ionenkanal-Rezeptor der GABA-Rezeptor ist.

19. Verfahren gemäß Anspruch 17 oder 18, wobei der Ionenkanal-Rezeptor ein Ionenkanal-GABA-Rezeptor ist, welcher aus der homomeren oder heteromeren Assoziation zwischen beliebigen GABA-Rezeptor-Untereinheiten resultiert, wobei die Untereinheit ausgewählt ist aus der Gruppe von GABA-Rezeptor-Untereinheiten bestehend aus alpha 1 bis 6-, beta 1 bis 3-, gamma 1 bis 3-, delta-, epsilon-, theta-, pi- und rho 1 bis 3-Untereinheiten, und die homomere oder heteromere Assoziation einen Ionenkanal-Rezeptor formt, welcher fähig ist, durch GABA aktiviert zu werden.

20. Verfahren gemäß Anspruch 18 oder 19, wobei der Agonist des Ionenkanal-Rezeptors GABA oder jeder andere gut bekannte GABA-Rezeptor-Agonist ist, wie Muscimol, Isoguvacine.

21. Kit zur Auswahl einer Verbindung, welche fähig ist, die Aktivität eines Ionenkanal-Rezeptors zu modulieren, wobei der Kit umfasst:
- Zellen welche rekombinant einen Ionenkanal-Rezeptor und einen P2X-Rezeptor co-exprimieren;
- einen Agonisten des P2X-Rezeptors, der ausgewählt ist aus der Gruppe bestehend aus ATP, α,β-mATP (alphabeta-Methylen-ATP), Benzoylbenzoe-ATP (wie 2'- und 3'-gemischte Isomere oder 2',3'-O-(4-Benzoylbenzoyl)-ATP (BzATP)) oder 2-Methylthio-ATP (2-MeSATP), vorzugsweise ATP; und
- eine Calciumsonde als einen Marker zur Messung des Calcium-Einstroms in den rekombinanten Zellen.

22. Kit gemäß Anspruch 21, wobei die Calciumsonde eine fluoreszierende Calciumsonde ist.

23. Kit gemäß Anspruch 21 oder 22, wobei der Kit ferner einen Agonisten des Ionenkanal-Rezeptors umfasst.

24. Kit gemäß Anspruch 23, wobei der Agonist des Ionenkanal-Rezeptors GABA ist.

25. Kit gemäß einem der Ansprüche 21 bis 24, wobei der Ionenkanal-Rezeptor, der P2X-Rezeptor, die rekombinanten Zellen und die Calciumsonde unabhängig ausgewählt werden unter den wie in den Ansprüchen 1 bis 20 definierten.

## Revendications

1. Méthode pour sélectionner ou identifier un composé capable de moduler l'activité d'un récepteur ionotrope, ladite méthode comprenant les étapes suivantes :
a) avoir des cellules co-exprimant de manière recombinante ledit récepteur ionotrope et un récepteur P2X ;
b) mettre en contact lesdites cellules recombinantes avec le composé à tester en présence d'un agoniste dudit récepteur P2X, ou en présence d'un agoniste dudit récepteur P2X et de l'agoniste dudit récepteur ionotrope ;
c) mesurer le courant calcique passant à travers le récepteur P2X, le courant calcique étant mesuré par une sonde calcique ;
d) comparer la mesure obtenue à l'étape c) avec la mesure obtenue dans les mêmes conditions au cours d'un premier test contrôle de référence, dans lequel le composé à tester n'est pas présent à l'étape b) ; et
e) sélectionner ledit composé si la comparaison de l'étape d) révèle une augmentation ou une diminution significative du courant calcique dans les cellules recombinantes en présence dudit composé.

2. Méthode selon la revendication 1, dans laquelle, à l'étape c), le courant calcique est mesuré par une sonde calcique fluorescente.

3. Méthode selon la revendication 1 ou 2, dans laquelle, à l'étape c), le courant calcique est mesuré par une sonde calcique fluorescente Calcium Green, Fluo-3 ou Fluo-4.

4. Méthode selon la revendication 1, dans laquelle, à l'étape a), lesdites cellules recombinantes n'expriment pas de manière endogène un récepteur capable d'être modulé par ledit agoniste dudit récepteur P2X ou par l'agoniste dudit récepteur ionotrope.

5. Méthode selon l'une quelconque des revendications 1 à 4, dans laquelle lesdites cellules recombinantes de l'étape a) sont des cellules de mammifère ou des ovocytes de Xénopus.

6. Méthode selon l'une quelconque des revendications 1 à 5, dans laquelle ledit récepteur P2X est un récepteur P2X naturel ou un récepteur P2X muté, capables d'interagir avec ledit récepteur ionotrope.

7. Méthode selon la revendication 6, dans laquelle ledit récepteur P2X est choisi dans le groupe constitué par les récepteurs P2X1, P2X2, P2X3, P2X4, P2X5, P2X6 ou P2X7 ou tout récepteur P2X résultant de l'association ou de la combinaison de sous-unités d'un récepteur P2X capable d'exercer une fonction de récepteur à canaux activable par l'ATP.

8. Méthode selon l'une quelconque des revendications 1 à 7, dans laquelle ledit récepteur ionotrope ou ledit récepteur P2X provient d'une source humaine, de rat ou de souris.

9. Méthode selon l'une quelconque des revendications 1 à 8, dans laquelle ledit agoniste dudit récepteur P2X est choisi dans le groupe constitué par l'ATP, l'α,β-mATP (alphabéta-méthylène-ATP), l'ATP benzoylbenzoïque (tel qu'un mélange des isomères 2' et 3' ou le 2',3'-O-(4-benzoylbenzoyl)-ATP (BzATP)), ou le 2-méthylthio-ATP (2-MeSATP), de préférence l'ATP.

10. Méthode selon l'une quelconque des revendications 1 à 9, dans laquelle ledit composé à tester est testé pour sa capacité à interagir spécifiquement avec ledit récepteur P2X, ladite interaction spécifique étant de préférence démontrée en mesurant si une augmentation ou une diminution significative du courant calcique est obtenue dans les mêmes conditions au cours d'un second test contrôle de référence, dans lequel les cellules recombinantes utilisées à l'étape a) expriment ledit récepteur P2X et n'expriment pas ledit récepteur ionotrope.

11. Méthode selon la revendication 10, dans laquelle ledit composé à tester n'est pas sélectionné si une augmentation ou une diminution significative du courant calcique est observée dans les cellules recombinantes au cours dudit second test contrôle de référence.

12. Méthode pour sélectionner ou identifier un agoniste d'un récepteur ionotrope, ladite méthode comprenant les étapes suivantes :
A) sélectionner ou identifier un composé capable de moduler l'activité dudit récepteur ionotrope par une méthode selon l'une quelconque des revendications 1 à 11 dans laquelle, à l'étape b), lesdites cellules recombinantes sont mises en contact avec le composé à tester uniquement en présence d'un agoniste dudit récepteur P2X ; et
B) sélectionner ledit composé comme agoniste si la comparaison réalisée à l'étape d) de la méthode selon l'une quelconque des revendications 1 à 11 révèle une diminution significative du courant calcique dans les cellules recombinantes en présence dudit composé par rapport aux résultats obtenus au cours du premier test de contrôle de référence.

13. Méthode pour sélectionner un modulateur positif d'un récepteur ionotrope, ladite méthode comprenant les étapes suivantes :
A) sélectionner ou identifier un composé capable de moduler l'activité dudit récepteur ionotrope par une méthode selon l'une quelconque des revendications 1 à 11 dans laquelle, à l'étape b), lesdites cellules recombinantes sont mises en contact avec le composé à tester en présence d'un agoniste dudit récepteur P2X et de l'agoniste dudit récepteur ionotrope ; et
B) sélectionner ledit composé comme modulateur positif si la comparaison réalisée à l'étape d) de la méthode selon l'une quelconque des revendications 1 à 11 révèle une diminution significative du courant calcique dans les cellules recombinantes en présence dudit composé par rapport aux résultats obtenus au cours du premier test de contrôle de référence.

14. Méthode pour sélectionner un modulateur positif d'un récepteur ionotrope selon la revendication 13, dans laquelle à l'étape b) de la méthode selon l'une quelconque des revendications 1 à 11 de l'étape A), l'agoniste dudit récepteur ionotrope est utilisé à une concentration non saturante.

15. Méthode pour sélectionner ou identifier un modulateur négatif, un inhibiteur ou un antagoniste d'un récepteur ionotrope, ladite méthode comprenant les étapes suivantes :
A) sélectionner un composé capable de moduler l'activité dudit récepteur ionotrope par une méthode selon l'une quelconque des revendications 1 à 11 dans laquelle, à l'étape b), lesdites cellules recombinantes sont mises en contact avec le composé à tester en présence d'un agoniste dudit récepteur P2X et de l'agoniste dudit récepteur ionotrope ; et
B) sélectionner ledit composé comme modulateur négatif si la comparaison réalisée à l'étape d) de la méthode selon l'une quelconque des revendications 1 à 11 révèle une augmentation significative du courant calcique dans les cellules recombinantes en présence dudit composé par rapport aux résultats obtenus au cours du premier test contrôle de référence.

16. Méthode selon l'une quelconque des revendications 1 à 15, dans laquelle les mêmes cellules recombinantes utilisées pour tester un premier composé peuvent être utilisées pour tester au moins un second composé à tester.

17. Méthode selon l'une quelconque des revendications 1 à 16, dans laquelle ledit récepteur ionotrope est choisi dans le groupe constitué par : le récepteur du GABA, le récepteur de la glycine, le récepteur de l'acétylcholine ou le récepteur de la sérotonine.

18. Méthode selon la revendication 17, dans laquelle ledit récepteur ionotrope est le récepteur du GABA.

19. Méthode selon la revendication 17 ou 18, dans laquelle ledit récepteur ionotrope est un récepteur ionotrope du GABA résultant de l'association homomérique ou hétéromérique de sous-unités du récepteur du GABA, ladite sous-unité étant choisie dans le groupe des sous-unités du récepteur du GABA constitué par : les sous-unités alpha 1 à 6, béta 1 à 3, gamma 1 à 3, delta, epsilon, théta, pi, et rho 1 à 3, ladite association homomérique ou hétéromérique formant un récepteur ionotrope capable d'être activé par le GABA.

20. Méthode selon la revendication 18 ou 19, dans laquelle l'agoniste dudit récepteur ionotrope est le GABA ou tout autre agoniste connu du récepteur du GABA, tel que le muscimol, l'isoguvacine.

21. Kit destiné à la sélection d'un composé capable de moduler l'activité d'un récepteur ionotrope, ledit kit comprenant :
- des cellules co-exprimant de manière recombinante un récepteur ionotrope et un récepteur P2X ;
- un agoniste du récepteur P2X choisi dans le groupe constitué par l'ATP, l'α,β-mATP (alphabéta-méthylène-ATP), l'ATP benzoylbenzoïque (tel qu'un mélange des isomères 2' et 3' ou le 2',3'-O-(4-benzoylbenzoyl)-ATP (BzATP)), ou le 2-méthylthio-ATP (2-MeSATP), de préférence l'ATP ; et
- une sonde calcique comme marqueur pour mesurer le courant calcique dans lesdites cellules recombinantes.

22. Kit selon la revendication 21, dans lequel ladite sonde calcique est une sonde calcique fluorescente.

23. Kit selon la revendication 21 ou 22, ledit kit comprenant en outre un agoniste dudit récepteur ionotrope.

24. Kit selon la revendication 23, dans lequel ledit agoniste dudit récepteur ionotrope est le GABA.

25. Kit selon les revendications 21 à 24, dans lequel ledit récepteur ionotrope, le récepteur P2X, les cellules recombinantes et la sonde calcique sont indépendamment choisis parmi ceux définis dans les revendications 1 à 20.
